(19) Europäisches Patentamt
European Patent Office
Office européen des brevets

(11) **EP 1 950 719 A2**

(12) **EUROPEAN PATENT APPLICATION**

(43) Date of publication:
**30.07.2008 Bulletin 2008/31**

(51) Int Cl.:
***G08B 21/02*** *(2006.01)*

(21) Application number: **07023009.9**

(22) Date of filing: **28.11.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **23.01.2007 CN 200710007350**

(71) Applicant: **Tsuen Shing Enterprises Limited
Hong Kong (HK)**

(72) Inventors:
• **Wong, Kent Hung Chit
Cheung Sha Wan
Kowloon
Hong Kong (HK)**

• **Kam, Suk Fun
Cheung Sha Wan
Kowloon
Hong Kong (HK)**
• **Peng, Bo
Cheung Sha Wan
Kowloon
Hong Kong (HK)**
• **Quan, Ci Kun
Cheung Sha Wan
Kowloon
Hong Kong (HK)**

(74) Representative: **Serjeants
25 The Crescent
King Street
Leicester, LE1 6RX (GB)**

(54) **Wireless monitoring system and method**

(57)    A wireless monitoring system, for example for monitoring infants, includes an infant terminal monitoring unit 10 and a parents terminal monitoring unit 12. The infant terminal monitoring unit 10 includes an image sensor module 14 and a microphone 18 for gathering image and voice information respectively, and associated modules for processing the gathered image and voice information. Gathered image and voice information is transmitted to the parents terminal monitoring unit 12 via a wireless data link, and the parents terminal monitoring unit 12 includes plurality of modules for processing the received image and voice information and a speaker 28 and a display screen 32 for relaying the received information.

Figure 1

**Description**

FIELD OF THE INVENTION

The invention relates to a wireless monitoring system. The invention also relates to a wireless monitoring method that uses said wireless monitoring system. The invention is particularly intended for use in the infant nursing field, and in particular provides a system and method for the real-time monitoring of infants.

BACKGROUND TO THE INVENTION

In modern society, people's desire for a good quality of life is increasing, and often there is a particular desire to spend more time taking care of their infants. However, due to the pace of today's life, it can be a challenge for people to find adequate time to look after their infants.

Infants cannot take care of themselves and thus require suitable monitoring and surveillance. Due to time pressures, it can, however, sometimes be difficult, impractical and inconvenient for many families to continuously monitor their infants, and this is especially the case for families with many infants.

It would therefore be desirable to provide a wireless monitoring system and method which address the abovementioned difficulties.

SUMMARY OF THE INVENTION

According to a first aspect of the present invention, there is provided a wireless monitoring system as defined in claim 1.

Optional but sometimes preferred features of the system are defined in claims 2 to 13.

According to a second aspect of the present invention, there is provided a wireless monitoring method as defined in claim 14.

Optional but sometimes preferred features of the method are defined in claims 15 to 22.

The wireless monitoring system and method according to the present invention may include one or more infant terminal monitoring units and one ore more parents terminal monitoring units. The master frame wireless data communication module of the or each infant terminal monitoring unit transmits gathered information or data (the two terms are used interchangeably in this specification) to the slave wireless data communication module of the one or more parents terminal monitoring units, and the system thus provides real-time dynamic monitoring of one or more infants.

The or each infant terminal monitoring unit sends image data and voice data gathered from an infant to the or each parents terminal monitoring unit via a wireless communication link after encoding the data, and the or each parents terminal monitoring unit relays the image data and voice data after decoding it. The system offers good reliability and is suitable for use by families and infant carers.

BRIEF DESCRIPTION OF DRAWINGS

Figure 1 is a function module sketch map of a wireless monitoring system according to the invention; and

Figure 2 is a working operation flow chart of a wireless monitoring system according to the invention.

DETAILED DESCRIPTION OF THE PREFERRED EMBODIMENT

In order to understand the technical content of this invention more clearly, the present invention is further exemplified by reference to the following non-limiting examples, which are provided for the purpose of illustration only.

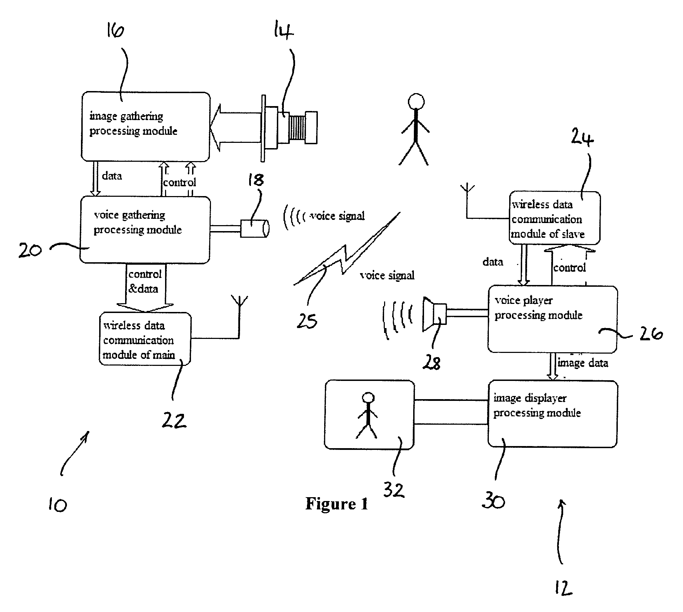

Referring to Fig. 1, the wireless monitoring system includes an infant terminal monitoring unit designated generally by the reference numeral 10 and a parents terminal monitoring unit designated generally by the reference numeral 12.

The infant terminal monitoring unit 10 includes an image sensor module 14, an image gathering and processing module 16, a microphone 18, a voice gathering and processing module 20, a main or master wireless data communication module 22, a main or master code-checking processing module (not shown) and a power management module (not shown) linked with each of the aforesaid modules. The image sensor module 14 may include a CMOS image sensor(s) or other type of image sensor(s).

The image sensor module 14 is linked with the main wireless data communication module 22 through the image gathering and processing module 16 and the voice gathering and processing module 20 in turn. The microphone 18 is linked with the voice gathering and processing module 20. The main code-checking processing module is linked with the main wireless data communication module 22.

In this example, the image gathering and processing module 16 and the voice gathering and processing module 20 can consist of a single module or multiple groups of modules.

In some embodiments, the image gathering and processing module 16 of the infant terminal monitoring unit 10 includes an image gathering main control unit, an image data gathering unit, an image data encoding unit and an image data storage unit. The image gathering main control unit is linked separately with each of the image sensor module 14, the image data gathering unit, the image data encoding unit and the image data storage unit.

The image data storage unit is linked with the voice gathering and processing module 20, which includes a voice gathering main control unit, a voice data sample gathering unit, a voice data encoding unit and a data package unit. The voice gathering main control unit is linked separately with each of the image data storage unit, the microphone 18, the voice data sample gathering unit, the voice data encoding unit and the data package unit separately.

The data package unit is linked with the main wireless data communication module 22. Additionally, the infant terminal monitoring unit 10 includes a main or master frame command processing module. The main command processing module is linked with the main wireless data communication module 22.

The parents terminal monitoring unit 12 includes a slave wireless data communication module 24, a voice player processing module 26, a speaker 28, an image display processing module 30, a display screen 32, a slave code-checking processing module (not shown) and a power management module (not shown) linked with each of the aforesaid modules. The display screen 32 can be screens with different pixels.

The slave wireless data communication module 24 is linked with the display screen 32 through the voice player processing module 26 and the image display processing module 30 in turn. The speaker 28 is linked with the voice player processing module 26. The slave code-checking processing module is linked with the slave wireless data communication module 24.

The main wireless data communication module 22 of the infant terminal monitoring unit 10 is linked with the slave wireless data communication module 24 of the parents terminal monitoring unit 12 by a wireless data link illustrated schematically by reference numeral 25.

In this example, the image display processing module 30 and the voice player processing module 26 can consist of a single module or multiple groups of modules.

In some embodiments, the voice player processing module 26 of the parents terminal monitoring unit 12 includes a voice player main control unit, a voice data decoding unit and a voice data player unit. The voice player main control unit is linked separately with each of the slave wireless data communication module 24, the voice data decoding unit and the voice data player unit. The voice data player unit is linked with the speaker 28. The voice data decoding unit is linked with the image display processing module 30.

The image display processing module 30 includes an image display main control unit, an image data decoding unit, an image data storage unit and an image data player unit. The image display main control unit is linked separately with each of the voice data decoding unit, the image data decoding unit, the image data storage unit and the image data player unit. The image data player unit is linked with the display screen 32.

Additionally, the parents terminal monitoring unit 12 includes a slave command processing module, which links with the slave wireless data communication module 24.

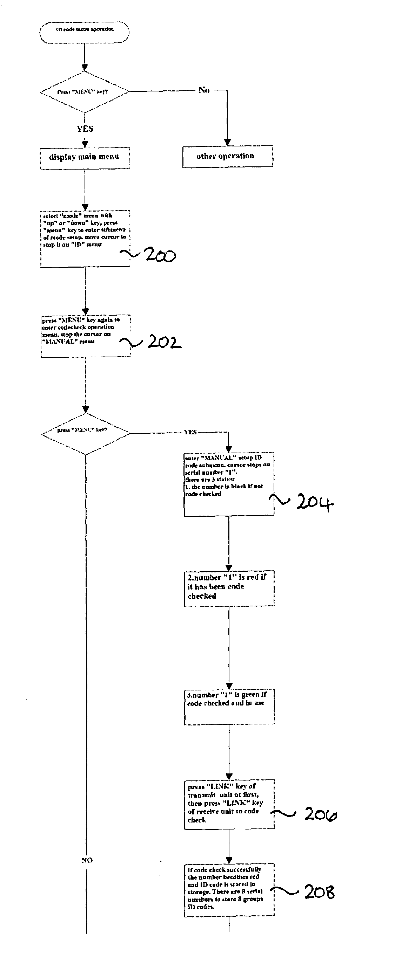

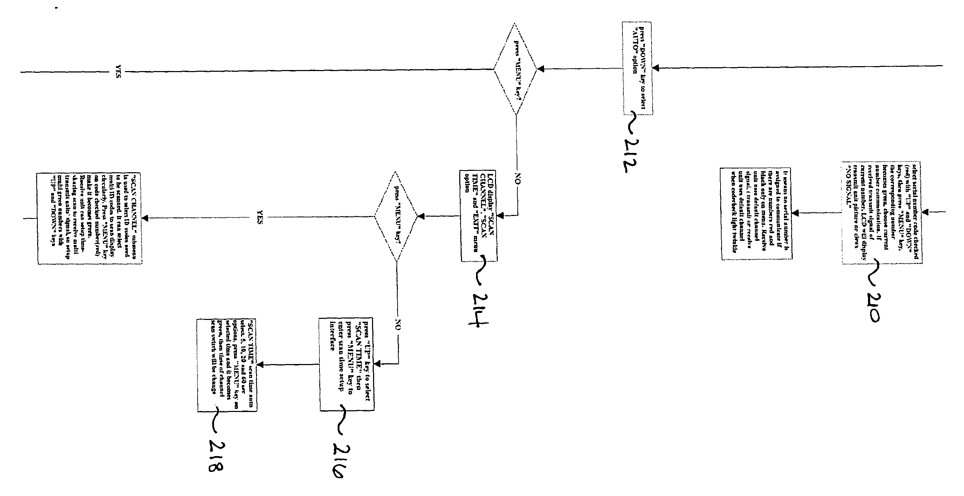

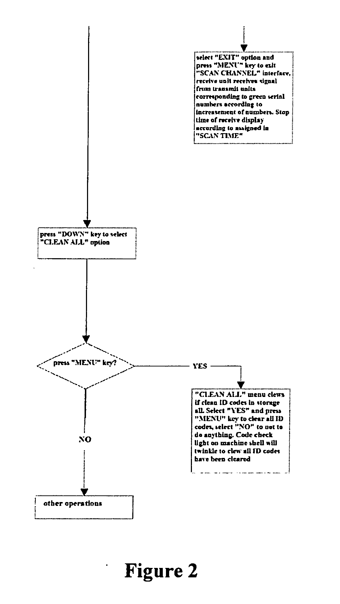

Referring now to Fig. 2, a method of effecting wireless monitoring using the system described above is described and includes following steps:

(1) Initialise the infant terminal monitoring unit 10 and the parents terminal monitoring unit 12;
(2) According to user operation, the system checks code between the infant terminal monitoring unit 10 and the parents terminal monitoring unit 12. This code-checking process includes the following steps:

(a) According to user operation, the main code-checking processing module of the infant terminal monitoring unit 10 produces a group code-checking information, including stochastic ID code, frequency-hopping group number and frequency-hopping sequence information;
(b) The main code-checking processing module stores the code-checking information;
(c) The main code-checking processing module sends the code-checking information to the parents terminal monitoring unit 12 through the main wireless data communication module 22 by wireless data signal;
(d) According to user operation, the parents terminal monitoring unit 12 receives the code-checking information through the slave wireless data communication module 24;
(e) The slave code-checking processing module stores the code-checking information;

(3) If the code-checking processing is successful, according to user operation, the parents terminal monitoring unit 12 continuously receives and relays in real-time monitoring information from the infant terminal monitoring unit 10, as follows:

(a) The infant terminal monitoring unit 10 continuously captures image information of a monitored infant using the image sensor module 14 and continuously captures voice information of the monitored infant using the microphone 18;

(b) The image gathering and processing module 16 gathers image data captured by the image sensor module 14 and sends this data to the voice gathering and processing module 20, the gathering and processing of the image information including the following steps:

(i) Gathering image data from the image sensor module 14 and analysing, encoding and storing the gathered data;

(ii) According to the image data gathered, determining a white balance parameter, an exposure parameter, and a gradation adjustment parameter of the image sensor module 14;

(iii) Controlling the image sensor module to obtain image data with a bright colour and high contrast;

(c) The voice gathering and processing module 20 gathers voice data sent by the microphone 18 and sends the image data and voice data to the main wireless data communication module 22 after encoding and packaging;

(d) According to the stochastic ID code, frequency-hopping group number and frequency-hopping sequence information, the main wireless data communication module 22 sends the data to the slave wireless data communication module 24 of the parents terminal monitoring unit 12;

(e) The voice player processing module 26 of the parents terminal monitoring unit 12 receives the data and decodes it, and sends the image data to the image display processing module 30;

(f) The image display processing module 30 decodes the image data and sends the decoded image data to the display screen 32 for display;

(4) According to user operation, the parents terminal monitoring unit 12 is set up to monitor multiple infant terminal monitoring units 10 automatically and sequentially, as follows:

(a) According to user operation, the system displays list of serial numbers of all the successfully code-checked infant terminal monitoring units 10;

(b) According to user operation, the system chooses infant terminal monitoring units 10 from the list to be monitored automatically and sequentially;

(c) According to user operation, the system displays list of scan time gaps for monitoring automatically and sequentially;

(d) According to user operation, the system chooses a scan time gap for monitoring automatically and sequentially from the list;

(5) According to setup information, the parents terminal monitoring unit 12 accepts and plays real-time monitoring information, as follows:

(a) the parents terminal monitoring unit 12 selects the first from the list of infant terminal monitoring units 10 to be monitored automatically and sequentially;

(b) the system accepts and plays real-time monitoring information from the infant terminal monitoring unit 10 and starts a timer, which includes following steps:

(i) the infant terminal monitoring unit 10 captures image information of a monitored infant using the image sensor module 16, and captures voice information of the monitored infant via the microphone 18;

(ii) the image gathering and processing module 16 gathers and processes image data sent by the image sensor module 14 and sends the data to the voice gathering and processing module 20. The gathering process includes following steps:

• Gathering image data from the image sensor module 14 for analysis, encoding and storage;
• According to the image data gathered, calculating a white balance parameter, an exposure parameter, and a gradation adjustment parameter of the image sensor module 14;
• Controlling the image sensor module 14 to obtain image data with bright colour and high contrast;

(iii) the voice gathering and processing module 20 gathers voice data sent by the microphone 18 and sends the image data and voice data to the main wireless data communication module 22 after encoding and packaging;

(iv) According to the stochastic ID code, frequency-hopping group number and frequency-hopping sequence information, the main wireless data communication module 22 sends the data to the slave wireless data communication module 24 of the parents terminal monitoring unit 12 using the frequency-hopping communication method;

(v) the voice player processing module 26 of the parents terminal monitoring unit 12 accepts the data for decoding and replay and sends the image data to the image display processing module 30;

(vi) the image display processing module 30 decodes the image data and sends it to the display screen 32 for display;

(c) If the timer exceeds the selected scan time gap, the system chooses the next one of the infant terminal monitoring units 10 to be monitored automatically and sequentially and returns to step (b);

(d) If all of the infant terminal monitoring units 10 have been chosen, the system chooses the first again and returns to step (b).

In a practical application, the code-checking process between the infant terminal monitoring unit 10 and the parents terminal monitoring unit 12 is as follows:

If there is no ID code in the infant terminal monitoring unit 10, the code-checking light will blink quickly. It will produce a group stochastic ID code, frequency-hopping group number and frequency-hopping sequence when a user presses the code-checking button on the infant terminal monitoring unit 10. They will be sent by high frequency and stored. After producing the ID code, the code-checking light will stop blinking, which means that the infant terminal monitoring unit 10 has been code checked. If the code-checking button is pressed again at this time, it will not produce a new ID code but will instead send the stored ID code unless the code-checking button is pressed for 3 seconds or more to clear the stored ID code. If there is no ID code in the parents terminal monitoring unit 12, the code-checking light will blink quickly. It will remain in a code-accepting state when pressing the code-checking button if the parents terminal monitoring unit 12 has entered the manual code-checking menu "MANUAL". It will store ID code when accept ID code in 1 to 5 seconds and the code-checking light will stop blinking. If you want to clear all ID codes, the parents terminal monitoring unit 12 enters "CLEAR ALL" menu, and select "YES", it will clear all ID codes and the code-checking light will blink to prompt none-code state again.

(1) infant terminal monitoring unit*1 + parents terminal monitoring unit*2 or more

For example: there is an infant terminal monitoring unit T1 and parents terminal monitoring units R1, R2 and R3. Before code-checking, R1 enters manual code-checking menu "MANUAL" first (steps 200 to 204), then press code-checking button of T1 once (step 206), then press code-checking button of R1 within 2 seconds (step 206), LCD of R1 will display information to prompt code-checking successful if code-checking process is successful (serial number selected by cursor becomes red) (step 208). Using to the above operating method, T1 can also be code checked with R2 and R3 respectively. After code-checking, choose serial numbers which are checked (red) in the "MANUAL" menu of parents terminal monitoring units and press the "MENU" button to make each serial number become green such that the parents terminal monitoring units receive data from the successfully code-checked infant terminal monitoring unit (step 210).

(2) infant terminal monitoring unit*2 or more + parents terminal monitoring unit* 1

For example: there are three infant terminal monitoring units T1, T2, T3 which need to communicate with a parents terminal monitoring unit R1. Turn on T1 and parents terminal monitoring unit R1, enter "MANUAL" menu of parents terminal monitoring unit R1 (steps 200 to 204) and choose a serial number, then press code-checking button to code check (step 206). Turn off T1 after finishing code-checking. Turn on T2, enter "MANUAL" menu (steps 200 to 204) of the parents terminal monitoring unit R1 and choose another serial number, then press code-checking button to code check (step 206). Turn off T2 after finishing code-checking. Repeat operation above to code check T3 and parents terminal monitoring unit R1. After finishing the preparation, turn on T1, T2 and T3, the parents terminal monitoring unit can choose the serial numbers code checked in the "MANUAL" menu of LCD to select to receive signal of T1, T2 or T3. The parents terminal monitoring unit also can choose ID codes for automatic scan switching mode with setting in "AUTO" menu (step 212). Depending on the selected time gap in "SCAN TIME" (steps 214 to 218), the parents terminal monitoring unit R1 switches a group ID code automatically to receive information from T1, T2 and T3 sequentially.

(3) infant terminal monitoring unit*2 or more + parents terminal monitoring unit*2 or more

For example: there are three infant terminal monitoring units T1, T2 and T3 and three parents terminal monitoring units R1, R2 and R3. In operation, R1 can switch to receive information from T1, T2 and T3; R2 can switch to receive information from T1 and T2; R3 can switch to receive information from T2, T3. This can be achieved by the code-checking operating method said above. First, turn on T1 and R1, press code-checking button to code check (steps 206 to 210), then code check T1 and R2 to finish the code-checking of T1. Second, turn on T2 and R1 to code check, T2 and R2 ...... Repeat these steps to finish the code-checking operation. After finishing all code-checking operations, turn on all infant terminal monitoring units T1, T2 and T3 and parents terminal monitoring units R1, R2 and R3. Parents terminal monitoring units R1, R2 or R3 can communicate with each other by selecting code-checked serial numbers through manual operating "MANUAL" menu, or by selecting ID code automatic switching mode using the "AUTO" menu (step 212) to receive information from T1, T2 or T3 sequentially, with switching a group ID code automatically in seconds.

The wireless monitoring method using the wireless monitoring system of this invention includes mainly the following functions: voice sample gathering function, voice player function, voice encoding and decoding function, high frequency module main controlling function, CMOS image sensor data gathering function, image data analysing processing function, CMOS image sensor main controlling function, data communication function.

It includes three MCU (main control units) controlling processors or modules as follows:

(1) CMOS image gathering and processing MCU: responsible for gathering image data from the CMOS sensors for analysis, encoding and storage. According to the gathered data, calculates a white balance parameter, an exposure parameter, and a gradation adjustment parameter for the CMOS sensors. Controls the CMOS sensors to obtain an image with bright colour and high contrast. Sends data to the next MCU.

(2) Voice gathering and processing MCU: responsible for gathering and encoding voice data, accepting image data from higher level MCU, getting user data. Encodes and packages the three data and sends it by controlling a 2.4G high frequency module. Uses 16K gather frequency in voice gathering, ADPCM compress, transfers two image data per second. 2.4G high frequency module uses frequency-hopping communication mode of 200 hoops/sec with 255 ID codes, three frequency groups with 16 frequency-hopping serials per group to avoid disturbing between transmitters. It also has a code-checking function, can produce stochastic ID code, frequency-hopping group number and frequency-hopping sequence information used to link again when code-checking.

(3) Voice player processing MCU: responsible for processing data received by 2.4G high frequency module. Decodes and analyses voice data and fill it in voice player alignment to play. Sends image data to next level MCU (SPL161001) for display. Stores received user data to supply the inquiry. Voice player uses velocity of 16K, with sound intensity indication function, volume control function. Controlling of high frequency module is the same as above.

(Optional) in functional description means this function is main body of function module, which must be run; (Optional) means this function is branch function convenient secondary developer. It can be decided by secondary developer to run it or not. Propose secondary developer not to process write operation to IOA register in program of main loop (because the main program will change the value of IOA in interrupt program), otherwise perhaps it will lead communication invalid (but read operation is no influence). If secondary developer wants to add IO control feet, use IOB port first. If it is used to be input port and unnecessary to process write operation to register in main loop program, then use left IO feet of IOA.

(I) MCU control CMOS function

Principle: Object of MCU controlling CMOS is colour image sensors, with resolution of $356\times292\times10$bit/dot in maximum. But this system only uses resolution of $216\times160\times4$bit/dot. There is 2KRAM only, not enough to store original data ($216\times160\times4$bit/dot/16bit/word=8640word) of a picture with $216\times160\times4$bit, so it is needed to expand a SRAM of 32K byte to store data.

In this function module, all IO ports of this sensor are occupied, and PWM output of TimerA is used as System Clock input (6MHz) of image sensor.

* 1 : IOA15 is controlled to send data of this module by mainframe MCU command
* 2 : IOB10 is data transfer port of this module, transfer data with UART protocol, linked with UART RX feet of MCU of main frame.

In this module, power of whole hardware module is controlled by low-level MCU (used in sleep control or electronic switch) to cut off power of module to save electricity in sleep or turn off (decided by secondary development). In this software module, any interrupt can not be opened absolutely (because the process of high speed gathering image data can not be interrupted), running time of the main program "void CMOS_CtrlandDataTransmit(void)" is about 0.5 second to complete four functions of CMOS controlling, CMOS image data gathering, image data processing, data transmission. Simply, it transfers data of an image running once.

Attention: secondary developer can run other program after calling main control program, but it will react speed of image transmission (two frames per second) if program running time is too long. Not to open the watchdog.

Function explanation:

(1) void MainInitializeForCMOS(void)

Function: CMOS gathers initial program of MCU (mandatory)
C format: void MainInitializeForCMOS(void)
Asm format: call _MainInitializeForCMOS
Input parameter: none
Return value: none
Attachment relationship: none
Occupied resource: UART, TimerA, IOAO-IOA15, IOBO-IOB15, not protect R1, R2, R3, R4, BP
Program period: ? command period

Explanation: initial all used RAM, function registers, IO ports, timers (TimerA PWM mode). This function need to be run only once after MCU on electricity, or called by secondary developer in program as replacement module.

Example program: example 1-1

(2) void CMOS_CtrlandDataTransmit(void)

Function: main control program of CMOS IC, including four functions about CMOS IC controlling, image gathering, image data processing, data transmission (mandatory)
C format: void CMOS_CtrlandDataTransmit(void)
Asm format: call _CMOS_CtrlandDataTransmit
Input parameter: none
Return value: none
Attachment relationship: need to run "void MainInitializeForCMOS(void)" function at least once after on electricity
Occupied resource: UART, IOA0-15, IOB0-15, not protect R1, R2, R3, R4, BP
Program period: ? command period
Explanation: This function processes gathering a picture and data transmission every time running, about 0.5 second. It can realize image output continuously and sequentially in main program.

Example program:

Ex 1-1:

For C language:

```
main()
 {
        MainInitializeForServe();        //run initialize program
                .
                .
        while(1)                         //main loop
        {
                .
                CMOS_CtrlandDataTransmit();  //call function in
 main loop to generate continuous images
```

```
          .
    }
        }
For ASM language
_main:
        call_MainInitializeForServe          //initialize
                    .
                    .
_Loop:
                    .
                call_CMOS_CtrlandDataTransmit         //sequentially
calling to realize continuous images
        jmp _Loop
```

(II) main frame controlling function

Principle: Objects controlled by main frame are: up-level CMOS controlling MCU, voice sample gathering, voice encoding, data package, 2.4G high frequency module. Up-level MCU is controlled to transfer data used to be packaged at what time. Voice sample is gathered with 16K@10bit sampling rate, and converted to voice data 64Kbps after compressed by ADPCM. Package and send voice data, image data, and command data according to emission period by high frequency module.

In mainframe control function, the most important factor is the accuracy of controlling of time sequence and rate of voice sample gathering of the high frequency module, and functions of time sequence controlling, voice sample gathering and data transmission are all realized in interrupt (the shortest interrupt gap is about 18us). So the secondary developer is advised to use setting flag bit method at interrupt (set flag bit in interrupt program and then exit interrupt program, enquiry from exterior program) to shorten running time of interrupt program to avoid influencing the main function (lead data missing). Because module function is mainly realized in interrupt, the main loop is no influential to the main function.

Main frame control function module need to wait about 0.5 second for power stableness after running initial function. Function modules work naturally after opening interrupt when power is stable. All kernel functions (voice sample gathering, data package, high frequency module control, master-slave code-checking) are finished in interrupt (16K FIQ Interrupt and UART Interrupt). It can work normally even run dead loop in main program (need to clear watchdog).

Main control function module further provides a function of code-checking between master and slave frame except main communication function. It will produce stochastic ID code, frequency-hopping group number and frequency-hopping sequence after code-checking and send them to slave frame by 2.4G high frequency module. This code-checking function is controlled by secondary developer. Module provides a code-checking start function, developer calls this function to start code- checking immediately. Time of master frame sending code-checking information is 3 second. It is finished automatically in interrupt by module without waiting. Developer can know current code-checking state by only calling code-checking state inquiry function. Master frame can't know whether slave frame received code-checking information because it is simplex communication between master and slave frames. So it needs master and slave frames start code-checking at the same time (press code-checking button) and approach as far as possible to avoid disturbing. Developer needs to get stochastic ID code, frequency-hopping group number and frequency-hopping sequence after code-checking and store them into storage in order to read out and write into module function next time to realize ID code memory.

In order to assist the secondary developer, main function module further opens 50 words data transfer protocol mostly used when master frame sends definite command data to slave frame.

Secondary developer needs to process encoding verification because the transferred data is not encoding protected. Developer should enter head address and data value of assigned data with command data before sending definite command data. Function module will enter data into sending queue automatically and send it at free time (longest delay is 0.5 second). Function module will send the command data at least once more at free time until new command data is entered. Function module provides inquiry function of command data sending state, developer can know if data is sent once or more with it. In view of data missing of wireless communication, developer is advised to reduce refresh frequency of command data (once per second or longer advised).

The IO port, FIQ interrupt with TimerA (16KHz), UART interrupt, software interrupt, ADC/DAC switch, MIC control occupied by this function module are advised not to be used. MIC circuit is attached in standard book.

* 1: used to control data transmission of up-level MCU, forbid when "1", allow when "0"
* 2: receive image data from up-level MCU with UART protocol

Function explanation:

(10void MainInitializeForMaster(void)

Function: initial program of main frame (mandatory)
C format: void MainInitializeForMaster(void)
Asm format: call _MainInitializeForMaster
Input parameter: none
Return value: none
Attachment relationship: none, initialize before main loop program
Occupied resource: software interrupt, FIQ+TimerA, UART, ADC, DAC, IOA0-2, IOA7-10, IOA12, IOB7, IOB10, not protect R1, R2, R3, R4, BP
Program period: ? command period
Explanation: initialize all used RAM of mainframe, function register, IO port, timer (TimerA 8K), call this function when on electricity, delay 0.5 second with software after running the function to wait for power to be stable, then open interrupt and enter main loop program Example program: example 2-1

Example 2-1:

For C language:

```
main()
{
        MainInitializeForMaster();  //run initial function when on electricity
        Delay();                              //software delay (developer writer)

                    .
                    .
        }
 For ASM language:
 _main:
      call _MainInitializeForMaster
      call _Delay
```

(2) void Sample_ForMaster(void)

Function: main frame voice sample gathering and RF main control program (mandatory)
C format: none
Asm format: call _SampIe_ForMaster
Input parameter: none
Return value: none
Attachment relationship: run "void MainInitializeForMaster(void)" function when initializing, open FIQ TimerA (8KHz) interrupt
Occupied resource: not protect R1
Program period: ? command period
Explanation: voice sample gathering and RF main control, must be called in 16KHz fast interrupt (FIQ) program. This function doesn't clear interrupt flag bit, it should be cleared by developer. Not to run other program within interrupt program and guarantee TimerA (16KHz) is the highest interrupt priority.

Example program: example2-2

Example 2-2:

For ASM language:

```
.text
.public _FIQ
_FIQ:
     push r1 to [sp]                    //R1 push stack protection
r1=0x3000
      [P_INT_Clear]=r1                  //clear interrupt flag bit
     call _Sample_ForMaster             //main control program
                     .
                     .
     pop r1 from [sp]                   //R1 pop stack recovery
reti
```

(3) void UART_TransmitForMaster(void)

Function: main control program of master frame UART function (mandatory)
C format: none
Asm format: call _UART_TransmitForMaster
Input parameter: none
Return value: none
Attached relationship: run "void MainInitializeForMaster(void)" function at initial, open UART interrupt
Occupied resource: not protect R1
Program period: ? command periods
Explanation: UART data transfer program, call UART interrupt program, clear UART launch automatically, receive flag bit, not to be interfered, not to run other programs in UART interrupt program to void interfering main function module

Example program: example 2-3

Example 2-3:

For ASM language:

```
.text
.public _IRQ7
_IRQ7:
     push r1 to [sp]
     call _UART_TransmitForMaster
     pop r1 from [sp]
reti
```

(4) void Start_RFCodeCheckForMaster(void)

Function: start master frame code-checking program (optional)
C format: void Start_RFCodeCheckForMaster(void)
Asm format: call _Start_RFCodeCheckForMaster
Input parameter: none
Return value: none
Attached relationship: initial function "void MainInitializeForMaster(void)" has been run, master frame has worked normally (FIQ (TimerA), IRQ7 (UART) interrupts are opened, main control program has run), used match with code-

EP 1 950 719 A2

checking state inquiry function "CodeCheck_Status(void)".
Occupied resource: not protect R1
Program period: ? command periods
Explanation: master frame code-checking time is 3 seconds, and it is finished in main control program automatically without software waiting by secondary developer. Master frame produces new stochastic ID code (value range of new ID code is 1-255), frequency-hopping group number and frequency-hopping sequence after starting code-checking, and sends them to slave frame by high frequency module. Master frame communicates with slave frame by new frequency-hopping parameter after finishing code-checking. Code-checking start function can be called in main loop of program only and must to be called again after finishing code-checking, otherwise code-checking fails.

Example program: example 2-4

(5) int CodeCheck_Status(void)

Function: inquiry program of code-checking state (optional)
C format: int CodeCheck_Status(void)
Asm format: call _CodeCheck_Status

$$[Stauts] = r1$$

Input parameter: none
Return value: 0x0000: no code-checking act, 0xffff: code-checking
Attached relationship: initial function "void MainInitializeForMaster(void)" has been run Occupied resource: not protect R1
Program period: ? command periods
Explanation: this function can be called by master and slave frames to inquire if acting code check, this function has no using limit after running initial function

Example program: example 2-4

Example 2-4 :

For C language :

```
      int i
main()
{
                    .
                    .
    while(1)                              //main loop
    {
    if(CodeCheckKeyDetect()==0xffff)     //test button state (written by developer)
if(Codecheck_Status()==0x0000)          //test code-checking act

Start_RFCodeCheckForMaster ();    //start code-checking program if there is no code-checking
act
                                    //if code-checking button is not pressed or code check
acting
//not to start code-checking and exit
                    .
    }
}
For ASM language :
_main:
.
```

11

```
        ·_Loop:
                        .
                        .
                        call_CodeCheckKeyDetect        //test button state (written
    by developer)
                        cmp r1,0xffff
                            jne NoCodeCheckKey   //jump out when no code check
    button pressed
                        call CodeCheck_Status        //inquire code-checking state
                        cmp r1,0x0000
                            jne NoCodeCheckKey //jump out when code check acting
                    call _Start_RFCodeCheckForMaster    //start when button pressed and no
    code check acting
    NoCodeCheckKey:
                        .
                        .
    jmp _Loop
```

(6) void CommandFillForMaster(int,int)

Function: master frame enters command data (optional)
C format: void CommandFillForMaster(Address,Sum)
Asm format : r1=_Sum //if it is variable, then it should be [_Sum]

```
    [sp--]=r1
    r1=_Address // if it is variable, then it should be [_Address]
    [sp--]=r1
    call _CommandFillForMaster
    sp+=2
```

Input parameter: Address is the head address of command data buffer memory assigned by secondary developer
Sum is the data amount necessary to be sent by secondary developer, value arrange is 0-50 Return value: none
Attached relationship: one dimensional array is assigned to be buffer of sending data by secondary developer, main function module has worked normally (main function has been initialized and interrupt has been opened)
Occupied resource: not protect R1
Program period: ? command periods
Explanation: command data is used to send some information of master frame to slave frame by secondary developer (for example: environment temperature of master frame, voltage of master frame's battery, master frame sleep, etc). Master frame will send command data in free time automatically and keep on sending the last command data to slave frame until new command data is entered. 50word data can be sent once. Command data is not verified and needs secondary developer to encode. It is advised to refresh command data once per second to insure slave frame receives complete data.

Example program: example 2-5

Example 2-5 :

For C language :

```
    int DataBuffer[50] ;                    //define data buffer, data amount is 50word
main()
        .
        .
{
    while(1)                                //main loop
    {
        .
```

```
                    if(TimingFor1Second()==0x0000)   //jump if less than 1 sec
                        {
                                .
                                UpDataForDataBuffer(); //refresh data in data buffer

                                CodingForDataBuffer();    //encode data in data buffer
                                CommandFillForMaster(DataBuffer,50);    //enter
   command data
                        }
                                                //not to refresh command data before 1 sec
            }
    }
```

For ASM language :

```
 .ram
 _DataBuffer:
 .dw 50 dup(0)                                    //define 50word, initial value is 0
 .code
 _ main:
 .
 .      _Loop:
                    .
                    .
                    call_TimingFor1Second         //1sec timer program
                    cmp r1,0x0000
                            jne NoTimeOut     //time less than 1sec, jump out
                    call _UpDataForDataBuffer   //refresh data in buffer
        call_CodingForDataBuffer             //encode buffer data
        r1=50                                //send 50word data
        [sp--]=r1
        r1=_DataBuffer                       //head address of data buffer
        [sp--]=r1
        call _CommandFillForMaster           //enter data command
                    .
                    .
 NoTimeOut:
 jmp _Loop
```

(III) slave frame control function

Principle: slave frame receives data from master frame by high frequency module and processes classified and automatically. Send voice data to play array after decoding. Send image data to image data play unit by UART TX port. Push command data into buffer waiting for taking out by secondary developer. Slave frame further controls power of image data play unit to realize standby sleep (decided by secondary developer). Slave frame decompresses and decodes received 64Kbps ADPCM data to play with 16KHz@10Bit.

Similar to master frame, main control functions of voice playing and high frequency module controlling are finished in interrupt program, so it needs to insure accuracy of FIQTimerA@16KHz interrupt and UART interrupt. It is advised to use flag bit method in interrupt program to reduce running time of interrupt program to insure communication between master and slave frames normally.

Function module provides code-checking function with master slave. Slave frame becomes zero ID code, zero frequency-hopping group, zero frequency serials after starting code-checking function and waits for code-checking command of master with frequency-hoop method. Slave frame exits code-checking state at once when kept on receiving correct ID code, frequency-hopping group, frequency serials in 1sec, or exits code-checking state automatically when not receiving correct ID code etc data in 5sec, and returns original ID code, frequency-hopping group, frequency serials. The ID code, frequency-hopping group, frequency serials need to be taken out by secondary developer and then stored into memory after code-checking.

Slave function module will refresh flag bit automatically to indicate receiving command data (command data receive inquiry function can be inquired). It can be inquired how much data received and taken. If command data is encoded on master frame, it needs to be decoded by developer after taking out from slave frame.

Function library further provides function sending command data to LCD control IC to control LCD display. It can send 20word data at most once and manifold circularly send, but it needs to advert running time of function. This function controls LCD display menu, icons expediently.

Slave frame function further provides some normal control functions for secondary developer facility, for details, see function explanation.

Working mode of slave frame is similar to master's, refer to master frame explanation.

The IO port, FIQ interrupt (6KHz) using TimerA, UART interrupt, software interrupt, DAC convert occupied by this function module can not to be used by secondary developer. Refer to standard book about power amplifier circuit or design it yourself.

*1 : linked to image data display unit, send image data to LCD to display

Function explanation :

(1) void MainInitializeForServe(void)

Function: initial program of slave frame (mandatory)
C format: void MainInitializeForServe(void)
Asm format: call _MainInitializeForServe
Input parameter: none
Return value: none
Attached relationship: none
Occupied resource : software interrupt, FIQ+TimerA, UART, DAC, IOA0-2, IOA7-10, IOB7,IOB10, not protect R1, R2, R3, R4, BP
Program period: ? command periods
Explanation: initialize all RAMs, function registers, IO ports, timers (TimerA 16K) used by master frame, call this function first after electrifying, then open interrupt and enter main loop program

Example program : example 3-1

Example 3-1 :

For C language :

```
main()
{
        MainInitializeForServe();        //initialize after electrifing
              .
              .
        while(1)                          //main loop
         {
              .
              .
}
    }
For ASM language :
_main:
     call_MainInitializeForServe // initialize after electrifing
              .
              .
 _Loop:                                   // main loop

              .
              .
      jmp  _Loop
```

(II) void Replay_ForServe(void)

Function: voice play and RF main control program of slave frame (mandatory)
C format: none
Asm format : call _Sample_ForServe
Input parameter: none
Return value: none
Attached relationship : run "void MainInitializeForServe(void)" function when initializing, open FIQ TimerA (16KHz) interrupt
Occupied resource: not protect R1
Program period: ? command periods
Explanation: voice play and RF main control needs to be called in 16KHz fast interrupt program (FIQ), this function does not clear flag bit, the developer should clear it by himself, not run other programs in interrupt program, and insure TimerA (16KHz) to be the highest interrupt priority

Example program: example 3-2

Example 3-2 :

For ASM language :

```
.text
.public _FIQ
_FIQ:
     push r1 to [sp]              //push stack protection
r1=0x3000
     [P_INT_Clear]=r1            //clear interrupt flag bit
     call _Sample_ForServe
              .
              .
     pop r1 from [sp]            //pop stack recovery
reti
```

(3) void UART_ReceiveForServe(void)

Function : function main control program of slave frame UART (mandatory)
C format : none
Asm format : call _UART_TransmitForServe
Input parameter : none
Return value : none
Attached relationship: run "void MainInitializeForServe(void)" function when initializing, open UART interrupt
Occupied resource: not protect R1
Program period : ? Command periods
Explanation: UART data transfer program, called in UART interrupt program, clear UART transmission by function automatically, receive flag bits, secondary developer need not to interfere, not to run other programs in UART interrupt program to avoid disturbing main function module

Example program : example 3-3

Example 3-3 :

For ASM language :

```
.text
.public _IRQ7
```

```
_IRQ7:
      push r1 to [sp]
      call _UART_TransmitForServe
      pop r1 from [sp]
reti
```

(4) void Start_RFCodeCheckForServe(void)

Function : start code-checking program of slave frame (optional)
C format : void Start_RFCodeCheckForServe(void)
Asm format : call _Start_RFCodeCheckForServe
Input parameter : none
Return value : none
Attached relationship : initial function "void MainlnitializeForServe(void)" has been run, slave frame works normally (FIQ (TimerA), IRQ7 (UART) interrupt opened, main control program has run), used match with inquiry function of code-checking state "CodeCheck_Status(void)"
Occupied resource : not protect R1
Program period : ? Command periods
Explanation : time of slave frame code-checking is 1-5sec (depends on time of receiving code check information), automatic time is accomplished in main control interrupt program, no need software waiting of secondary developer. Slave frame starts with zero ID code, zero frequency-hopping group, zero frequency serials after starting code-checking, and waits for code-checking information from master frame. Code-checking starting function is called in program main loop only and called again after code-checking finish, otherwise code-checking fails.

Example program : example 3-4

(5) int CodeCheck_Status(void)

Function: inquiry program of code-checking state (optional)
C format: int CodeCheck_Status(void)
Asm format : call _CodeCheck_Status

$$[Stauts] = r1$$

Input parameter : none
Return value : 0x0000 : no code check action, 0xffff : code check acting
Attachment relationship : initial function "void MainlnitializeForServe(void)" has run
Occupied resource : not protect R1
Program period : ? Command periods
Explanation : this function can be called by master and slave frames to inquire if code-checking is acting, this function has no using limit after running initial function

Example program : example 3-4

Example 3-4 :

For C language :

```
main()
{
        .
        .
```

```
        while(1)
  if(CodecheckKeyDetect()-0xfff)                //test button state
  if(CodeCheck_Status()==0x0000)                //test if code check acting
  Start_RFCodeCheckForServe();          //start code check program if no code check action
                                        //no code check button pressed or code check acting,
                                            //not to start code check and exit


      .

}
 For ASM language:
 _main:
.
.

       _Loop:
             .
             .
                 call_CodeCheckKeyDetect                  //inquire button state
                 cmp r1,0xffff
                     jne NoCodeCheckKey       //jump out with no code check
 button pressed
                 call CodeCheck_Status        //inquire code check status
                 cmp r1,0x0000
                     jne NoCodeCheckKey //code check acting, jump out
          call _Start_RFCodeCheckForServe   //start when button is pressed and
 code check not acting
 NoCodeCheckKey:
                  .
                  .
 jmp _Loop
```

(6) int CommandRxInqForServe(void)

    Function: slave frame inquires if received command data
    C format: int CommandRxInqForServe(void)
    Asm format : call _CommandRxInqForServe


$$[Status]=r1$$


    Input parameter: none
    Return value : 0x0000 : received data, 0xffff : not received data
    Attachment relationship: main function module has worked normally (main function has been initialized and has opened interrupt)
    Occupied resource: not protect R1
    Program period: ? Command periods
    Explanation: slave frame will refresh this flag bit when received command data every time, secondary developer can know command data is received and data amount of received command data is also known, then read data to assigned buffer

Example program : example 3-5


(7) int CommandRxInqGrossForServ(void)

    Function: slave frame inquires data amount of received command data
    C format: int CommandRxInqGrossForServe(void)

Asm format : call_CommandRxlnqGrossForServe

$$[\_Gross]=r1$$

Input parameter: none
Return value: received data amount, 0-50 (50 is the most amount)
Attachment relationship : main function module has worked normally (main function has been initialized and opened interrupt)
Occupied resource: not protect R1
Program period: ? Command periods
Explanation: secondary developer can get data amount of received command data from this function, and read out data to assigned buffer

Example program : example 3-5

(8) void CommandFetchForServe(int,int)

Function : slave frame reads out received command data
C format : void CommandFetchForServe(Address,Sum)
Asm format : r1=Sum //if it is variable, become [_Sum]

```
[sp--]=r1
r1=Address // if it is variable, become [_Address]
[sp--]=r1
call _CommandFetchForServe
```

Input parameter : Address is head address of buffer assigned by secondary developer to receive command data

Sum is data amount read out by secondary developer, the most is 50

Return value : none
Attachment relationship : it is necessary for secondary developer to assign a one-dimension array to be buffer for sending data, main function module has worked normally (main function has been initialized and has opened interrupt)
Occupied resource : not protect R1
Program period : ? Command periods
Explanation : secondary developer reads out data to assigned buffer with is function. If data is encoded on main, slave frame needs to decode data after taken out

Example program : example 3-5

Example 3-5 :

For C language :

```
int I;
int DataBuffer[50];
main()
{
    .
    .
    while(1)
    {
        .
        .
```

```
        if(CommandRxInqForServe()==0x0000)              //inquire if received data
        {
                i=CommandRxInqGrossForServe();          //inquire data amount
received
                CommandFetchForServe(DataBuffer,i);     //read out data to buffer
                DeCodingForDataBuffer();                     //decode data
                DataFunction();                         //realize data function
        }
        .
        .
    }
}
```

For ASM language :

```
.ram
_DataBuffer:
.dw 50 dup(0)                              //define 50word RAM space
.code


_main:
    _Loop:
                .
            .
            call _CommandRxInqForServe   //inquire if received data
            cmp r1,0x0000
                    jne NoDataReceive    //jump out if no data received
        call _CommandRxInqGrossForServe  //get data amount to store in R1
        [sp--]=r1                        //input data amount
        r1=_DataBuffer                   //input head address of buffer
        [sp--]=r1
        call_CommandFetchForServe
        sp+=2                                 //recover stack
        call _DeCodingForDataBuffer          //decode data
        call _DataFunction                   //realize data function
    NoDataReceive :
            .
            .
        jmp _Loop
```

(9) void LCDCommandForServe(int,int)

Function : slave frame sends command data to LCD displayer (optional)
C format : void LCDCommandForServe(Address,Sum)
Asm format : r1=[_Sum] //send data sum

    [sp--]=r1
    r1=[_Address] //buffer address of data needs to be sent
    [sp--]=r1

call _LCDCommandForServe
input parameter : Address : head address of data (array)

    Sum : data sum sent

Return value : none
Attachment relationship : initial function "void MainInitializeForServe(void)" has been run
Occupied resource : not protect R1
Program periods : run 5ms mostly

Explanation : slave frame sends command data to LCD controller, send once with calling this function once, running time of function is 5ms mostly. Command data uses word as unit, but command data can't have byte alignment sequence as below : 0x00 -> 0xff -> 0xff -> 0xa0 (or 0x0f). command data needs to be validated by user.

Example program : example3-6

Example 3-6 :

```
    main()
    {
            ......
            if(InqNeedTransmitLCDComm()==0xffff)        //user write, inquire if
command data send
            {
                    LCDCommandForServe(Address,Sum);    //send command data
    }
        }
```

(10) int RFReceiveStatusForServe(void)

Function : slave frame inquires receiving status (optional)
C format : int RFReceiveStatusForServe(void)
Asm format : call _RFReceiveStatusForServe

$$[Status]=r1$$

Input parameter : none
Return value : 0x0000 : receive normally, 0xffff : signal interrupt
Attachment relationship : initial function "void MainInitializeForServe(void)" has been run, slave frame has worked normally (FIQ (TimerA), IRQ7 (UART) interrupt have opened, main control program is running)
Occupied resource : not protect R1
Program period : ? Command periods
Explanation : slave frame can get current receiving quality when calling this function, and act turn on/off voice play

Example program : example 3-7

(11) void Start_VoiceReplay(void)

Function : allow slave frame to play voice (optional)
C format : void Start_VoiceReplay(void)
Asm format : call _Start_VoiceReplay
Input parameter : none
Return value : none
Attachment relationship : initial function "void MainInitializeForServe(void)" has been run, slave frame has worked normally (FIQ (TimerA), IRQ7 (UART) interrupt has opened, main control program is running)
Occupied resource : not protect R1
Program period : ? Command periods
Explanation : turn on voice play, voice data enters DAC buffer
Example program : example 3-7

(12) void Stop_VoiceReplay(void)

Function : turn off slave frame voice play (optional)
C format : void Stop_VoiceReplay(void)
Asm format : call _Stop_VoiceReplay
Input parameter : none
Return value : none
Attachment relationship : initial function "void MainlnitializeForServe(void)" has been run, slave frame has worked normally (FIQ (TimerA), IRQ7 (UART) interrupt has opened, main control program is running)
Occupied resource : not protect R1
Program period : ? Command periods
Explanation : stop voice play, DAC no output

Example program : example 3-7

Example 3-7 :

For C language:

```
Int Volume;              /define volume value storage
Int VoiceLevel;       //define sound intensity value storage
main()
{
            .

while(1)
 {
            .
            .
    if(RFReceiveStatusForServe()==Ox0000)        //inquire receiving status
            Start_VoiceReplay();        //start voice play when receiving
    Else
            Stop_VoiceReplay();        //stop voice play when no receiving
    Volume=KeyForVolume();                   //control volume key
    SetVolumeLeve(Volume);                   //change volume
        .
        .
 }
}
```

For ASM language:

```
.ram
.var _Volume                          // define volume value storage
.var _VoiceLevel       // define sound intensity value storage
.code
_main:
            .
            .
_Loop:
            .
            .
        call _RFReceiveStatusForServe     // inquire receiving status
        cmp r1,0x0000
            jne NoReceive               // stop voice play when no receiving
        call_Start_VoiceReplay          // start voice play when receiving
        jmp Normal
 NoReceive:
        Call _Stop_VoiceReptay
```

```
    Normal:

    Call_KeyForVolume                    // control volume key
    [_Volume]=r1                         //output volume value
    r1=[_Volume]                         //input volume value parameter
    Call _SetVolumeLeve                  //change volume
                          .
                          .
                jmp _Loop
```

(IV) Master/Slave general function

The following functions are generally used to store ID code. After initialization, the master and slave machine both set the ID code, frequency group, frequency-hopping sequence to 0. Those data are modified after master and slave code-checking procedure. The secondary developer needs to store those parameters to the storage to ensure the master and salve machine to work as a pair after reboot. 93C46 can be used as storage, or use flash memory of 061A during development phase, dependent on the secondary developer's decision.

There is another special usage of these functions: Slave machine can store multiple frequency-hopping parameters (ID code, frequency group, frequency-hopping sequence). And the slave machine can switch from one parameter group to another (use Load_XXX function) from one period (i.e. 5 second) to let the slave machine (Act as a monitor) to scan multiple master machines (CCD camera).

For example: there are four master machines with the following frequency-hopping parameters:

| Machine No | ID Code | Frequency Group | Freq-Hopping Seq. |
|---|---|---|---|
| #1 | 198 | 2 | 8 |
| #2 | 20 | 0 | 10 |
| #3 | 245 | 2 | 2 |
| #4 | 67 | 1 | 14 |

Slave machine uses code-checking or other methods (decided by the secondary developer) to obtain the frequency-hopping parameters of the four master machines. First, fill frequency-hopping parameters of master #1 into slave machine, the slave machine scans master #1 to establish communication, and receive data from machine #1; after 5s (the time is decided by secondary developer), then fill frequency-hopping parameters of master #2 into slave machine, the slave machine communicates with master #2, again after 5s, the slave machine communicates with master #3 etc. and so on to realize scan function (corresponding to channel scan function of monitor).

Function explanation:

(1) int Catch_IDcode(void)

Function : Get the ID code from master machine (optional)

C format : int Catch_IDCode(void)

Asm format : call _Catch_IDCode

$$[ID\_Code]=r1$$

Input parameter : none

Return value : ID code current using, value range: 0-255

Attachment relationship : Main function is running

Occupied resource : not protect R1

Program period: ? command periods

Explanation: ID code is a random number generated in the code-checking process, after initialized in the main function, the ID code becomes zero, it is necessary to read out ID code from storage and fill in.

Example program : example 4-1

(2) void Load_IDCode(int)

Function : Change the ID code currently using (Optional)
C format : void Load_IDCode(ID_Code)
Asm format : r1=ID_Code // [ID_Code] if is variable
call _Load_IDCode
Input parameter : needed ID code, value range: 0-255
Return value : none
Attachment relationship : Main function is running
Occupied resource : not protect R1
Program period: ? command periods
Explanation: This function are mainly used by secondary developer to read out ID code from storage to send to module to use

Example program : example 4-1

(3) int Catch_FreBank(void)

Function: Get current frequency group code from master machine (Optional)
C format : int Catch_FreBank(void)
Asm format : call _Catch_FreBank

$$[Fre\_Bank]=r1$$

Input parameter : none
Return value : The current frequency group number, value range: 0-2
Attachment relationship : Main function is running
Occupied resource : not protect R1
Program period: ? command periods
Explanation: Frequency group number is a random number generated in code code-checking process. The frequency group number becomes zero after initializing main function. Need to read out frequency group number from storage to fill in.

Example program : example 4-1

(4) void Load_FreBank(int)

Function: Change current frequency group number of the machine (Optional)
C format : void Load_FreBank(Fre_Bank)
Asm format : r1=Fre_Bank // [Fre_Bank] if is variable call _Load_FreBank
Input parameter : needed frequency group number, value range: 0-2

Return value : none
Attachment relationship : Main function is running
Occupied resource : not protect R1
Program period: ? command periods
Explanation: This function is mainly used by secondary developer to read out frequency group number from the storage to module to use

Example program : example 4-1

(5) int Catch_ChSeNum(void)

Function: Get the current frequency-hopping sequence from the master machine (Optional)
C format: int Catch_ChSeNum(void)
Asm format: call _Catch_ChSeNum

$$[ChSe\_Num]=r1$$

Input parameter: none
Return value: The current frequency-hopping sequence, value range: 0-15
Attachment relationship : Main function is running
Occupied resource : not protect R1
Program period: ? command periods
Explanation : Frequency-hopping sequence is a random number generated in code-checking process. The frequency-hopping sequence becomes zero after initializing main function. Need to read out frequency-hopping sequence from storage to fill in.

Example program : example 4-1

(6) void Load_ChSeNum(int)

Function: Change the current frequency-hopping sequence (Optional)
C format: void Load_ChSeNum(ChSe_Num)
Asm format: r1=ChSe_Num // [ChSe_Num] if is variable call _Load_ ChSeNum
Input parameter: The current frequency-hopping sequence, value range: 0-15
Return value: none
Attachment relationship: Main function is running
Occupied resource: not protect R1
Program period: ? command periods
Explanation: This function is mainly used by secondary developer to read out frequency-hopping sequence from storage to module to use

Example program: example 4-1

(7) void SetFrequencyHopping(void)

Function: use frequency-hopping to communicate
C format: void SetFrequencyHopping(void)
Asm format: call_SetFrequencyHopping
Input parameter: none
Return value: none

Attachment relationship: Program initialized
Occupied resource: not protect R1
Program period: ? command periods
Explanation: Set the frequency-hopping on. Can be called anywhere in the program, take effective immediately.

Example program: example 4-1

(8) void SetFrequencySingle(int)

Function: Set the frequency-hopping off, using single frequency
C format: void SetFrequencySingle(Channel_Number)
Asm format: r1=[_Channel_Number]

$$[sp--]=r1$$

call _SetFrequencySingle
sp+=1
Input parameter: Channel_Number: The frequency channel number to use (0-94)
Return value: none
Attachment relationship: Program initialized
Occupied resource: not protect R1
Program period: ? command periods
Explanation: Set the frequency-hopping off. Can be called anywhere in the program, take effective immediately.

Example program: example 4-1

(9) int VoiceLevel_Detect(void)

Function: Sound intensity detecting program (Optional)
C format: int VoiceLevel_Detect(void)
Asm format: call _VoiceLevel_Detect

$$[Level]=r1$$

Input parameter: none
Return value: Current sound intensity level, value range: 0x0-0x1ff (Hex number), the higher the level, the higher the sound intensity
Attachment relationship: initial function "void MainInitializeForMaster/Serve(void)" has been run, main function has worked properly (FIQ (TimerA), IRQ7 (UART) Interrupt is on, main controlling program is running)
Occupied resource: not protect R1
Program period: ? command periods
Explanation: sound intensity detecting function gets voice sampling or play sound intensity. It can be used in sound control detection or drive of sound intensity indicator light.

Example program: example 4-1

Example 4-1 :

For C language:

```
int IDCode;                // Define ID Code storage
```

```
        int FreBank;                    //Define frequency group No. storage
        int ChSeNum;                        //Define frequency-hopping sequence storage
        int Flag=0x0000;                            //Define flag
        main()
        {

                MainInitializeForXXXXX();        //initialize function module
                IDCode=ReStoreIDCode();          //get ID code from storage
                FreBank=ReStoreFreBank();        //get frequency group No. from storage
                ChSeNum=ReStoreChSeNum ();        //get frequency-hopping sequence No.
    from storage
                Load_IDCode(IDCode);                 //fill in ID code
                Load_FreBank(FreBank);               //fill in frequency group No.
                Load_ChSeNum(ChSeNum);    //fill in frequency-hopping sequence No.
    SetFrequencyHopping();              //use frequency-hopping to communicate
                            .
                            .
                while(1)
                {
                            .
                            .
    if(Codecheck_Status()==0x0000)       //check if code-checking is in progress
            Flag=0xffff;                          //set flag for starting code-checking
                    if(Flag==0xffff)              //check the flag for starting code-checking
            if(CodeCheck_Status()==0xffff)        //check if code-checking is not in progress
                {
                        Flag=0x0000;                 //clear the flag
                        IDCode=Catch_IDCode();        //Get ID code after code-checking
                        FreBank=Catch_FreBank();      //Get frequency group No. after code-
    checking
                        ChSeNum=Catch_ChSeNum();      //Get frequency-hopping No. after
    code-checking
                        StoreIDCode(IDCode);       //store into storage
                        StoreFreBank(FreBank);     //store into storage
                        StoreChSeNum(ChSeNum);     //store into storage
                         }
            VoiceLevel=VoiceLevel_Detect();          //Get current sound intensity value
            LEDDirve(VoiceLevel);                 //Drive LED using sound intensity value
                            .
                            .


                }
        }
        For ASM language:
        .ram
    .var _IDCode;                            // Define ID Code storage
        .var _FreBank;                               //Define frequency group No. storage
        .var _ChSeNum;                       //Define frequency-hopping sequence No. storage
        .var _Flag=0x0000;                           //Define Flag
        .code
        _main:
                call _MainInitializeForXXXXX         //initialize function module
                call _ReStoreIDCode                           //get ID code from storage
                [_IDCode]=r1
                call _ReStoreFreBank        //get frequency group No. from storage
                [_FreBank]=r1
                call_ReStoreChSeNumBank         //get frequency-hopping sequence No.
    from storage
                [_ChSeNum]=r1
                r1=[_IDCode]
                call _Load_IDCode                         //fill in ID code
                r1=[_FreBank]
                call_Load_FreBank                 //fill in frequency group number
```

```
                r1=[_ChSeNum]
                call_Load_ChSeNum        //fill in frequency-hopping sequence No.
                          .
                          .
_Loop:
                          .
                          .
call_CodeCheck_Status          //check if code-checking is in process
cmp r1,0x0000
jne NoCodeCheck                           //if no code-checking, jump out
[_Flag]=0xffff                            //set code-checking start flag

NoCodeCheck:
                R1=0xffff
                cmp r1,[_Flag]                    //inquire code-checking start flag
                        jne CodeCheckNotStart     //if code-checking is not started,
jump out
                call_CodeCheck_Status      //check if code-checking is not in process
                cmp r1,0x0000
                        jne CodeCheckNotStart      //code-checking is not finished
                r1=0x0000
[_Flag]=r1;                                       //clear the flag after code-checking
                call _Catch_IDCode                //get ID code after code-checking
                [_IDCode]=r1
                call _Catch_FreBank       //get frequency group No. after code-checking
                [_FreBank]=r1
                call _Catch_ChSeNum       //get frequency-hopping sequence No. after code-
checking
                [_ChSeNum]=rl
                r1=[_IDCode]
                call_StoreIDCode                  //store into storage
                r1=[_FreBank]
                call_StoreFreBank                 //store into storage
                rl=[_ChSeNum]
                call_StoreChSeNum                 //store into storage
CodeCheckNotStart:
Call_VoiceLevel_Detect                    //Get current sound intensity value
                          .
                          .
[_VoiceLevel]=r1
r1=[_VoiceLevel]                          //Input sound intensity value parameter
call_LEDDirve                             //Drive LED using sound intensity value
                jmp _Loop
```

(V) Power supply management function

Functions below are fit for testing battery voltage with ADC function or other ADC applications. ADC function is dependent on TimerA, so TimerA interrupt is to be ensured opening when ADC function is running.

Function explanation :

(1) void SetADCScanPin(int)

```
Function: assign ADC channel needed to be used
C format: void SetADCScanPin(int PinNum)
Asm format: r1=[_PinNum]
call _SetADCScanPin
Input parameter: PinNum: assign Pin port needed to be ADC scanned, (value range 0x0-0x6 means IOA0~6)
Return value: none
```

Attachment relationship : none

Occupied resource : not protect R1

Program period : ? Command periods

Explanation : This function sets a single ADC channel needed to be scanned (corresponding to IOA port), setup attribution of scanned IO port in function. Setup one ADC channel once time. If want to setup multi ADC channels, call this function times. This function should be called at initializing.

Example program : example 5-1

(2) void ADCScan(void)

Function: ADC function main management program

C format: void ADCScan(void)

Asm format: call _ADCScan

Input parameter: none

Return value: none

Attachment relationship : main program has been initialized, TimerA is running

Occupied resource : not protect R1

Program period : ? Command periods

Explanation : this function is used to scan and get data of ADC channels (channels scanned must be initialize defined by calling void SetADCScanPin(int) function before using this function), function running time is: 1ms×sum of channel. Function should be called in main loop. Scan all ADC channels needed to be scanned every time once calling, and refresh values of all ADC channels immediately.

Example program : example 5-1

(3) void ADCResult(void)

Function: get value of ADC channel

C format: int ADCResult(int PinNum)

Asm format: r1=[_PinNum]

call _ADCScan

$$[\_ADCValue]=r1$$

Input parameter: PinNum: assign ADC channel needed to get value (0-6: IOAO-6)

Return value: 12bit ADC convert value of corresponding ADC channel (bit0~bit11 are effective)

Attachment relationship : void ADCScan(void) has run normally

Occupied resource : not protect R1

Program period : ? Command periods

Explanation : this function is used to get sample value of ADC channels scanned, available when lower than 12bit. If PinNum is not assigned to be ADC scanned, the return value is invalid. User judges by himself.

Example program : example 5-1

Example 5-1 :

```
Main()
{
        int ADC7,ADC6;
```

```
MainInitializeForMaster();          //main initialize program
.
.

SetADCScanPin(6);                                      //set scan ADC channel 7(IOA.6)
SetADCScanPin(5);                                      //set scan ADC channel 6(IOA.5)
IRQ_ON(FIQ_TMA);
while(1)
{
.
.

ADCScan();                          //ADC scans sample gathering program

              ADC7=ADCResult(6);          //get ADC value of ADC7 (IOA.6)
              ADC6=ADCResult(5);          //get ADC value of ADC7 (IOA.5)
              Clear_WDT();
                               .
}
}
```

There is thus provided a wireless monitoring system and method including an infant terminal monitoring unit 10 linked with a parents terminal monitoring unit 12, and in which the infant terminal monitoring unit 10 establishes wireless communication with the parents terminal monitoring unit 12 through the main wireless data communication module 22 linked with the slave wireless data communication module 24. The infant terminal monitoring unit 10 sends gathered image data and voice data of an infant to the parents terminal monitoring unit 12 through a wireless communication link after an encoding process, and the parents terminal monitoring unit 12 relays the image data and voice data after decoding it, to provide real-time dynamic monitoring of an infant. This system with stable working performance and widely applicable scope is fit for families and infant nursing. It adds convenience to people's lives and lays solid foundations for the further development of infant nursing techniques.

Although embodiments of the invention have been described in the preceding paragraphs with reference to various examples, it should be appreciated that various modifications may be made to the examples without departing from the scope of the invention, as claimed.

## Claims

**1.** A wireless monitoring system, including:

an infant terminal monitoring unit (10) comprising:

an image sensor module (14) and an image gathering and processing module (16);
a voice gathering and processing module (20) and a microphone (18) linked with the voice gathering and processing module (20);
a master frame wireless data communication module (22) and a master frame code-checking processing module linked with the master frame wireless data communication module (22);
a power management module linked with each of the aforesaid modules;

wherein the image sensor module (14) is linked with the master frame wireless data communication module (22) in turn via the image gathering and processing module (16) and the voice gathering and processing module (20);
a parents terminal monitoring unit (12) comprising:

a voice player processing module (26) and a speaker (28) linked with the voice player processing module (26);
an image display processing module (30) and a display screen (32);
a slave frame wireless data communication module (24) and a slave frame code-checking processing module linked with the slave frame wireless data communication module (24);
a power management module linked with each of the aforesaid modules;

wherein the slave frame wireless data communication module (24) is linked with the display screen (32) in turn via the voice player processing module (26) and the image display processing module (30);

and wherein the master frame wireless data communication module (22) of the infant terminal monitoring unit (10) is arranged to communicate in use with the slave frame wireless data communication module (24) of the parents terminal monitoring unit (12) via a wireless data link.

2. A wireless monitoring system according to claim 1, wherein the display screen is linked with the image display processing module.

3. A wireless monitoring system according to claim 1 or claim 2, wherein the image gathering and processing module includes an image gathering main control unit, an image data gathering unit, an image data encoding unit and an image data storage unit, and wherein the image gathering main control unit is linked separately with each of the image sensor module, the image data gathering unit, the image data encoding unit and the image data storage unit respectively, and wherein the image data storage unit is linked with the voice gathering and processing module.

4. A wireless monitoring system according to claim 3, wherein the voice gathering and processing module includes a voice gathering main control unit, a voice data gathering unit, a voice data encoding unit and a data package unit, and wherein the voice gathering main control unit is linked separately with each of the microphone, the image data storage unit, the voice data gathering unit, the voice data encoding unit and the data package unit respectively, and wherein the data package unit is linked with the master frame wireless data communication module.

5. A wireless monitoring system according to any preceding claim, wherein the infant terminal monitoring unit further includes a master frame command processing module, and the master frame command processing module is linked with the master frame wireless data communication module.

6. A wireless monitoring system according to any preceding claim, wherein the image sensor module includes a CMOS image sensor.

7. A wireless monitoring system according to any preceding claim, wherein the image gathering and processing module and the voice gathering and processing module consist of a single module.

8. A wireless monitoring system according to any of claims 1 to 6, wherein the image gathering and processing module and the voice gathering and processing module consist of multiple modules.

9. A wireless monitoring system according to any preceding claim, wherein the voice player processing module includes a voice player main control unit, a voice data decoding unit and a voice data player unit, the voice player main control unit being linked separately with each of the slave frame wireless data communication module, the voice data decoding unit and the voice data player unit, and wherein the voice data player unit is linked with the speaker and the voice data decoding unit is linked with the image display processing module.

10. A wireless monitoring system according to any preceding claim, wherein the image display processing module includes an image display main control unit, an image data decoding unit, an image data storage unit and an image data player unit, and wherein the image display main control unit is linked separately with each of the voice data decoding unit, the image data decoding unit, the image data storage unit and the image data player unit, and wherein the image data player unit is linked with the display screen.

11. A wireless monitoring system according to any preceding claim, wherein the parents terminal monitoring unit further includes a slave frame command processing module, and wherein the slave frame command processing module is linked with the slave frame wireless data communication module.

12. A wireless monitoring system according to any preceding claim, wherein the image display processing module and the voice player processing module consist of a single module.

13. A wireless monitoring system according to any of claims 1 to 11, wherein the image display processing module and the voice player processing module consist of multiple modules.

14. A wireless monitoring method using the system according to any preceding claim, wherein the method includes the following steps:

(i) initialising the infant terminal monitoring unit (10) and the parents terminal monitoring unit (12);
(ii) code-checking between the infant terminal monitoring unit (10) and the parents terminal monitoring unit (12);
(iii) if the code-checking is successful, continuously receiving information from the infant terminal monitoring unit (10) at the parents terminal monitoring unit (12) and continuously relaying the received information in real-time via the parents terminal monitoring unit (12).

**15.** A method according to claim 14, wherein the parents terminal monitoring unit receives information sequentially from a plurality of infant terminal monitoring units and sequentially relays the received information in real-time via the parents terminal monitoring unit.

**16.** A method according to any preceding claim, wherein one or more parents terminal monitoring units receive information from one or more infant terminal monitoring units and relay the received information in real-time.

**17.** A method according to any of claims 14 to 16, wherein during the step of code-checking between the infant terminal monitoring unit and the parents terminal monitoring unit:

(i) the master frame code-checking processing module of the infants terminal monitoring unit produces group code-checking information;
(ii) the master frame code-checking processing module stores the code-checking information;
(iii) the master frame code-checking processing module sends the code-checking information through the master frame wireless data communication module via the wireless data link to the parents terminal monitoring unit;
(iv) the parents terminal monitoring unit receives the code-checking information via the slave frame wireless data communication module;
(v) the slave frame code-checking processing module stores the received code-checking information.

**18.** A method according to claim 17, wherein the code-checking information includes stochastic ID code, frequency-hopping group number and frequency-hopping sequence information.

**19.** A method according to claim 15 or any of claims 16 to 18 when ultimately dependent on claim 15, wherein the method includes the step of setting up the parents terminal monitoring unit to receive information sequentially from the plurality of infant terminal monitoring units, and during said step the system:

(i) displays a list of the plurality of code-checked infant terminal monitoring units and their respective associated serial numbers;
(ii) sequentially selects and sets up the plurality of infant terminal monitoring units from the displayed list;
(iii) selects a scan time gap for sequentially receiving information from the plurality of infant terminal monitoring units.

**20.** A method according to claim 19, wherein:

(i) the parents terminal monitoring unit selects the first infant terminal monitoring unit from the displayed list;
(ii) the parents terminal monitoring unit receives and relays in real-time information from the infant terminal monitoring unit selected in step (i) and starts a timer;
(iii) when the timer exceeds the selected scan time gap, the parents terminal monitoring unit selects the next infant terminal monitoring unit from the displayed list and re-executes step (ii);
(iv) when the final infant terminal monitoring unit has been selected from the displayed list, the parents terminal monitoring unit re-executes steps (i) to (iv).

**21.** A method according to any of claims 14 to 20, wherein during the step of receiving information from the infant terminal monitoring unit at the parents terminal monitoring unit and continuously relaying the received information in real-time via the parents terminal monitoring unit:

(i) the infant terminal monitoring continuously captures image information and voice information from an infant using the image sensor module and the microphone respectively;
(ii) the image gathering and processing module gathers and processes the captured image information received from the image sensor module and transmits the processed image information and captured voice information to the voice gathering and processing module;
(iii) the voice gathering and processing module gathers the voice information captured by the microphone, encodes and packages the image information and the voice information, and transmits the encoded and packaged image and voice information to the master frame wireless data communication module;

(iv) according to the stochastic ID code, frequency-hopping group number and frequency-hopping sequence information, the master frame wireless data communication module transmits the packaged image and voice information to the slave frame wireless data communication module of the parents terminal monitoring unit using a frequency-hopping mode;

(v) the voice player processing module of the parents terminal monitoring unit receives and decodes the packaged voice information, plays it via the speaker, and transits the image information to the image display processing module;

(vi) the image display processing module decodes the image information and displays it on the screen.

22. A method according to claim 21, wherein the step of gathering and processing the image information captured by the image sensor module comprises:

(i) gathering image information from the image sensor module and analysing, encoding and storing said gathered image information;

(ii) determining a white balance parameter, an exposure parameter, and a gradation adjustment parameter of the image sensor module;

(iii) controlling the image sensor module so that the gathered image information has a bright colour and high contrast.

**Figure 1**

ID code menu operation

Press "MENU" key? — No → other operation

YES

display main menu

select "mode" menu with "up" or "down" key, press "menu" key to enter submenu of mode setup. move cursor to stop it on "ID" menu ~200

press "MENU" key again to enter codecheck operation menu, stop the cursor on "MANUAL" menu ~202

press "MENU" key? — YES →

enter "MANUAL" setup ID code submenu. cursor stops on serial number "1". there are 3 status: 1. the number is black if not code checked ~204

2.number "1" is red if it has been code checked

3.number "1" is green if code checked and in use

press "LINK" key of transmit unit at first, then press "LINK" key of receive unit to code check ~206

If code check successfully the number becomes red and ID code is stored in storage. There are 8 serial numbers to store 8 groups ID codes. ~208

NO

```
                              ┌─────────────────────────┐
                              │ select serial number code checked │
                              │ (red) with "UP" and "DOWN" │
                              │ keys, then press "MENU" key, │
                              │ the corresponding number │ ～ 210
                              │ becomes green, choose current │
                              │ number communication. If │
                              │ received transmit signal of │
                              │ current number, LCD will display │
                              │ transmit unit picture or clews │
                              │ "NO SIGNAL" │
                              └─────────────────────────┘
                                        │
                                        ▼
                              ┌─────────────────────────┐
                              │ It means no serial number is │
                              │ assigned to communicate if │
                              │ there are numbers red and │
                              │ black only on menu. Receive │
                              │ unit uses default channel │
                              │ signal. ( transmit or receive │
                              │ unit uses default channel │
                              │ when codecheck light twinkle │
                              └─────────────────────────┘
```

press "DOWN" key to select "AUTO" option  ～ 212

press "MENU" key? — NO → LCD display "SCAN CHANNEL", "SCAN TIME" and "EXIT" menu option ～ 214

YES (left side)

press "MENU" key? — NO → press "UP" key to select "SCAN TIME" then press "MENU" key to enter scan time setup interface ～ 216

YES

"SCAN TIME" scan time auto select. 5, 10, 20 and 60 sec options, press "MENU" key on selected time and it becomes green, then time of channel scan switch will be change ～ 218

"SCAN CHANNEL" submenu is used to select ID codes need to be scanned. It can select multi ID codes to scan display circularly. Press "MENU" key on code checked number(red) make it becomes green. Receive unit can setup time-sharing scan to receive multi transmit units' signal, so setup multi green numbers with "UP" and "DOWN" keys

35

select "EXIT" option and
press "MENU" key to exit
"SCAN CHANNEL" interface,
receive unit receives signal
from transmit units
corresponding to green serial
numbers according to
increasement of numbers. Stop
time of receive display
according to assigned in
"SCAN TIME"

press "DOWN" key to select
"CLEAN ALL" option

press "MENU" key?                    YES

                                     "CLEAN ALL" menu clews
                                     if clean ID codes in storage
                                     all. Select "YES" and press
                                     "MENU" key to clear all ID
                                     codes, select "NO" to not to
                                     do anything. Code check
                                     light on machine shell will
        NO                           twinkle to clew all ID codes
                                     have been cleared

other operations

# Figure 2